# EUROPEAN PATENT APPLICATION

(11) **EP 4 837 587 A1**
(43) Date of publication of application: **11.11.2026**
(21) Application number: 25174564.2
(22) Date of filing: 06.05.2025
(51) Int. Cl.: A61K 39/09, C07K 14/315, C12N 15/62

(54) **IMMUNOGENIC FUSION PROTEIN**

(71) Applicant: MinervaX ApS, 2000 Frederiksberg (DK)
(72) Inventor: FISCHER, Per, 2000 FREDERIKSBERG (DK); JOHANSSON LINDBOM, Bengt, 224 57 LUND (SE)
(74) Representative: Öberg & Sjöberg AB

(57) **Abstract**

The proposed technology relates to an immunogenic fusion protein comprising or consisting of an amino acid sequence consisting of: i. a first amino acid sequence part consisting of 170 to 178 amino acids, preferably 174 to 175 amino acids, and having at least 90% sequence identity with the amino acid sequence shown in SEQ ID NO: 7; ii. a second amino acid sequence part consisting of 170 to 178 amino acids, preferably 174 to 175 amino acids, and having at least 90% sequence identity with the amino acid sequence shown in SEQ ID NO: 7; and optionally: iii. a linker amino acid sequence part consisting of 1 to 20 amino acids and separating the first amino acid sequence part from the second amino acid part. The immunogenic fusion protein preferably consists of 340 to 376 amino acids, preferably 348 to 370 amino acids, more preferably 349 to 351 amino acids. The proposed technology further pertains to nucleic acid molecule encoding the immunogenic fusion protein; a vector; a host cell; a vaccine; and a use of any of the foregoing in a vaccination against, or treating, a group B streptococcus infection.

## Description

### TECHNICAL FIELD

The technology proposed herein relates to the fields of microbiology and vaccine technology and concerns the development of an immunogenic fusion protein capable of conferring immunity to group B Streptococcus infections. More particularly, the technology proposed herein relates to an immunogenic fusion protein which confers immunity to invasive strains of the group B Streptococcus. It further pertains to a nucleic acid molecule encoding the immunogenic fusion protein; a vector; a host cell; a vaccine; and a use of any of the foregoing in a vaccination against, or treating, a group B streptococcus infection.

### BACKGROUND

Group B Streptococcus *(Streptococcus agalactiae)* (GBS) is the major cause of invasive bacterial infections, including meningitis, in the neonatal period. In the United States alone, there are now about 5000 cases per year of invasive disease caused by this bacterium. These infections have an overall mortality of about 10%, and many of the infants that survive have permanent neurological sequelae. In view of this, a large effort has been made to find methods of prevention and treatment and to analyze the mechanisms by which GBS cause infections.

GBS can also cause mastitis in cows, a bovine disease that is of considerable economical importance. Development of a vaccine against GBS infections is therefore of interest also in veterinary medicine.

About 20 % of all women are vaginal carriers of GBS, and vertical transmission from the maternal genital tract is probably the most common source of infection in neonatal disease caused by this bacterium. However, only about 1 % of the infants that are colonized by the GBS at birth are afflicted by serious infection. Other factors than exposure to the bacterium during birth must therefore contribute to the development of neonatal disease.

Group B streptococcal strains are divided into nine serotypes (Ia, Ib, and II-VIII) based on the structure of the polysaccharide capsule. The four "classical" serotypes Ia, Ib, II, and III occur in roughly equal proportions among strains in the normal flora, but type III is the clinically most important serotype, in particular because it causes most cases of meningitis. Because the capsule is a known virulence factor, it has been studied in considerable detail, in particular in type III strains. Efforts have been made to develop a vaccine, in which the type III polysaccharide capsule would be an essential component.

EP 0 866 133 discloses a vaccine capable of protecting a recipient from infection caused by group B *Streptococcus.* The proposed technology is directed to the use of a combination of a polysaccharide and a fragment of the epsilon protein. It further discloses that epidemiological data suggest that the type-specific capsule plays an important role in the immunity to group B *Streptococcus* infections (see page 7 line 2-3). Additionally, there are a number of different combinations between different proteins and the polysaccharide mentioned within the application, but all the claims comprise a polysaccharide which shows the importance of that particular component. However, use of the polysaccharide capsule as a vaccine may give problems due to cross reactions with human tissues (Pritchard et al., Infect Immun 1992. 60: 1598). It would therefore be very valuable if one could develop a vaccine based on proteins rather than on polysaccharides.

The document Gravekamp et al., Infection and Immunity, Dec 1997, p 5216-5221 discloses the evaluation of the immunogenicity as well as protection of the number of repeats of the alpha (α) C protein as well as the N-terminal part alone. It was found that the immunogenicity decreased with increasing number of repeats (se Fig 2B). However, it was also found in a protection assay that the antibodies against the N-terminal region were predominantly responsible for the protection compared to antibodies against the repeat regions (see page 5219 left column, line 6 from the bottom, and page 5220 right column lines 26-29).

WO 9410317 describes the use of the alpha protein, a GBS surface protein, in the development of a conjugate vaccine. A drawback with this protein is that it usually is not expressed by type III strains, which are the cause of many serious GBS infections. Hence, a protective immunity against these strains will not be evoked by an alpha protein vaccine.

WO 9421685 describes the use of the Rib protein, a GBS surface protein, in the development of a vaccine. This protein elicits immunity when administered with alum. However, the Rib protein has the disadvantage that it does not evoke a protective immunity against all GBS strains.

The document Lindahl et al, Nonimmunodominant Regions Are Effective As Building Blocks In A Streptococcal Fusion Protein Vaccine, Cell Host & Microbe 2, 427-434, December 2007, discloses a fusion protein comprising N-terminal regions of the group B Streptococcus surface proteins Rib and Alpha C (herein AlpC).

Similarly, WO 2008127179 describes a fusion protein comprising at least one first N-terminal region fragment of a group B Streptococcus surface protein or analogue, homologue, derivative or immunologically related amino acid sequence or fragments thereof, which is fused to at least one second N-terminal region fragment of a group B Streptococcus surface protein or analogue, homologue, derivative or immunologically related amino acid sequence or fragments thereof, wherein the first and second at least one N-terminal region fragments of group B Streptococcus surface proteins derive from different group B Streptococcus strains, and wherein the fusion protein is capable of eliciting protective immunity against group B Streptococcus.

WO 2022207657 describes a fusion protein comprising at least one first N-terminal region fragment of a group B Streptococcus protein, Rib which is fused to at least one second N-terminal region fragment of a group B Streptococcus surface protein, AlpC.

Despite the advances in the progress towards a vaccine suitable for prevention of GBS disease, there is still a need for further methods and vaccines for prevention and treatment of GBS infections. Thus, there remains a need to explore vaccines strategies capable of eliciting protective immunity against a wide range of GBS strains.

Accordingly, it is a primary objective of the technology proposed herein to provide an immunogenic fusion protein which can be used in a vaccine capable of eliciting protective immunity against GBS infections.

It is a further object of the proposed technology to provide a nucleic acid molecule comprising a polynucleotide encoding the immunogenic fusion protein.

Further objects of the technology proposed herein include the provision of a vector comprising the nucleic acid molecule, a host cell expressing the immunogenic fusion protein or comprising the nucleic acid molecule or the vector,
Yet further objects of the technology proposed herein include the provision of a vaccine as well as the use of any of the foregoing in vaccination against group B Streptococcus infection, or treating a group B Streptococcus infection.

The means of accomplishing each of the above objectives as well as others will become apparent from the description of the proposed technology which follows hereafter.

### SUMMARY OF THE PROPOSED TECHNOLOGY

The present technology is based on further work, by the present inventor, in relation to the immunogenic fusion proteins disclosed in WO 2022207657, such fusion proteins comprising at least one first N-terminal region fragment of a group B Streptococcus Rib protein which is fused to at least one second N-terminal region fragment of a group B Streptococcus AlpC protein. In such a fusion protein, the immunogenicity of the Rib N terminal region fragment has been found to be unexpectedly reduced in comparison to the immunogenicity of the non-Rib N-terminal region fragment. Compared with the fact that a majority of GBS strains express Rib, it revealed a need to increase the immune response towards Rib. By producing a fusion protein comprising two Rib N terminal region fragments, the reduction of the immune response against Rib may be mitigated. Specifically, an immunogenic fusion protein with both the first and second amino acid sequence comprising an amino acid sequence of the N-terminal region of Rib is expected to be suitable to elicit a strong Rib immune response. Such an immunogenic fusion protein has a higher efficacy in eliciting a RibN immunogenic response, and overcomes the weak RibN competitiveness in stimulating B-cells compared to other fusion protein comprising only one N terminal region of Rib.

Another advantage with the immunogenic fusion protein is that it can be combined with a further immunogenic fusion protein. Such a further immunogenic fusion protein may comprise the N-terminal regions of Rib and AlpC (referred to as GBS-NN or GBS-NN1). Another such further immunogenic fusion protein may comprise the N-terminal regions of Alp1 and Alp2 (referred to as GBS-NN2). Thus, in a vaccine comprising either/or both of GBS-NN and GBS-NN2 fusion protein/ proteins it may be advantages to add the RibN-RibN immunogenic fusion protein herein to increase the immunogenicity towards Rib. Furthermore, the desired level of immunogenicity towards Rib is easily adjusted by modifying the amount of RibN-RibN fusion proteins in the vaccine compositions containing e.g. RibN-AlpCN and/or Alp 1N-Alp2N.

Additionally, codon optimized corresponding nucleic acid molecules encoding the immunogenic fusion protein have been devised. These may be provided in a vector. A host cell may in turn express the immunogenic fusion protein or comprise the vector. Each of the immunogenic fusion protein, the nucleic acid molecule, the vector, and the host cell may be used in a vaccine. The immunogenic fusion protein, the nucleic acid molecule, the vector, the host cell, and/or the vaccine may further be used in a method of vaccination against group B Streptococcus infection or treating a group B Streptococcus infection.

Thus, a first aspect of the proposed technology relates to an immunogenic fusion protein comprising or consisting of an amino acid sequence consisting of:
i. a first amino acid sequence part consisting of 170 to 178 amino acids, preferably 174 to 175 amino acids, and having at least 90% sequence identity with the amino acid sequence shown in SEQ ID NO: 7;
ii. a second amino acid sequence part consisting of 170 to 178 amino acids, preferably 174 to 175 amino acids, and having at least 90% sequence identity with the amino acid sequence shown in SEQ ID NO: 7; and optionally:
iii. a linker amino acid sequence part consisting of 1 to 20 amino acids and separating the first amino acid sequence part from the second amino acid part,
and wherein the immunogenic fusion protein preferably consists of 340 to 376 amino acids, preferably 348 to 370 amino acids, more preferably 349 to 351 amino acids.

As noted above, the immunogenic fusion protein has a higher efficacy in Rib immunogenicity for use as a vaccination. The immunogenic fusion protein has the advantage that it is immunogenic even without adjuvant, however it can also be used with an adjuvant such as alum or Aluminum hydroxide (AlOH) for increased immunogenicity.

The proposed technology will be described in more detail below.

### DETAILED DESCRIPTION OF THE PROPOSED TECHNOLOGY

In this specification, unless otherwise specified, "a" or "an" means "one or more". Throughout the specification, any and all references are specifically incorporated into this patent application by reference.

As above, a first aspect of the proposed technology relates to an immunogenic fusion protein comprising or consisting of an amino acid sequence consisting of:
i. a first amino acid sequence part consisting of 170 to 178 amino acids, preferably 174 to 175 amino acids, and having at least 90% sequence identity with the amino acid sequence shown in SEQ ID NO: 7;
ii. a second amino acid sequence part consisting of 170 to 178 amino acids, preferably 174 to 175 amino acids, and having at least 90% sequence identity with the amino acid sequence shown in SEQ ID NO: 7; and optionally:
iii. a linker amino acid sequence part consisting of 1 to 20 amino acids and separating the first amino acid sequence part from the second amino acid part,
and wherein the immunogenic fusion protein preferably consists of 340 to 376 amino acids, preferably 348 to 370 amino acids, more preferably 349 to 351 amino acids.

The term "immunogenic" is intended to mean having the ability to elicit an immune response. The immunogenic fusion protein of the proposed technology is immunogenic and characterized by its ability to elicit a protective immune response against at least GBS containing the surface proteins of which the N-terminal regions are comprised by the immunogenic fusion protein. Eliciting protective immunity against group B Streptococcus is to be understood as encompassing eliciting protective immunity against group B streptococcus carrying at least the surface protein Rib. "Capable" means that the immunogenic fusion protein, if administered in a suitable dose to an individual having an immune system, elicits protective immunity in that individual. The immunogenic fusion protein is preferably capable of eliciting protective immunity against group B Streptococcus.

The term "protective immunity" in relation to the proposed technology refers to the ability of serum antibodies and/or cytotoxic T cell response induced during immunization to protect (partially or totally) against disease caused by an infectious agent, such as a group B Streptococcus. That is, a vertebrate immunized by the vaccines of the proposed technology will experience limited growth and spread of group B Streptococcus. To determine whether protective immunity is induced by a fusion protein or vaccine, techniques well known for a person skilled in the art can be used. For example, to determine whether immunization with a fusion protein or vaccine of the proposed technology induces protective immunity against group B Streptococcus infection, immunized test animals can be challenged with group B Streptococcus and growth and spread of the group B Streptococcus is measured.

For the purpose of the proposed technology the term "fusion protein" refers to an assembly of two or more protein regions, or fragments thereof. As stated above, the immunogenic fusion protein comprises (or preferably consists) of an amino acid sequence consisting of at least first and second amino acid sequence parts. Both of these parts are related to the N-terminal region of the group B Streptococcus surface protein Rib.

The combination of amino acid sequence parts to obtain a fusion protein can be accomplished by fusing, i.e., joining, the amino acid sequence parts together at one or both ends thereof (covalent coupling, bond, or linking, or conjugation), or by joining together separate nucleic acid molecules encoding the respective amino acid sequence parts, such that a single continuous expression product is produced when the joined nucleic acid molecules are translated.

In a fusion protein, a first and a second amino acid sequence part may be separated by a linker amino acid sequence part.

It is further contemplated within the context of the proposed technology that a fusion protein can be obtained by cross-linking the amino acid sequence parts or by capturing them on a macromolecular structure.

For the purpose of the proposed technology the term "protein" refers to a molecular chain of amino acids. A protein is not of a specific length and can, if required, be modified in vivo or in vitro, by, for example, glycosylation, amidation, carboxylation or phosphorylation. Inter alia, peptides, oligopeptides and polypeptides are included within the definition.

The term "N-terminal region" in relation to the proposed technology refers to an N-terminus region (N) of a protein.

Group B streptococcus strains, also referred herein as GBS, are known and may be isolated from the blood of infected human beings. GBS is the most common cause of neonatal sepsis in the United States and is responsible for about 5000 cases per year.

The denotation "Group B streptococcus" derives from the fact that Streptococci have been divided into immunological groups based upon the presence of specific carbohydrate antigens on their cell surfaces. At present, groups A through O are recognized.

The group B Streptococcus Rib protein, also referred to in this specification as Rib and Rib protein, is a surface protein known in the art, and for example described in WO 9421685. The denotation "Rib" refers to: Resistance to proteases, immunity, and group B. The Rib protein was first isolated from a group B streptococcal strain of serotype III as a distinct 95 kDa protein. Protein Rib is expressed by almost all group B streptococcal strains of the clinically important serotype III, which cause most cases of meningitis, and by some strains of other serotypes such as II. Moreover, Rib is expressed by all strains of a hypervirulent clone of type III. A method has been devised to purify protein Rib and it has been demonstrated that antibodies to this protein protect against lethal infection with strains expressing protein Rib (for further details, such as DNA and protein sequences see WO 9421685).

SEQ ID NO: 7 shows the N-terminal Region of Rib:
MAEVISGSAVTLNTNMTKNVQNGRAYIDLYDVKNGKIDPLQLITLNSPDLK
AQYVIRQGGNYFTQPSELTTVGAASINYTVLKTDGSPHTKPDGQVDIINVSL
TIYNSSALRDKIDEVKKKAEDPKWDEGSRDKVLISLDDIKTDIDNNPKTQSD
IANKITEVTNLEKILVPRIP

Nucleic acid DNA molecules encoding the amino acid sequence of SEQ ID NO: 7 are shown in SEQ ID NOs: **1,** 3, 5, where SEQ ID NO: 3 is codon optimized for expression in *E. coli,* and SEQ ID NO: 5 is codon optimized for expression in *Cricetulus griseus* (Chinese Hamster Ovary) cells.

Corresponding nucleic acid RNA molecules encoding the amino acid sequence of SEQ ID NO: 7 are shown in SEQ ID NOs: 2, 4, 6, where SEQ ID NO: 4 is codon optimized for expression in *E. coli,* and SEQ ID NO: 6 is codon optimized for expression in *Cricetulus griseus* (Chinese Hamster Ovary) cells.

The term "sequence identity" indicates a quantitative measure of the degree of homology between two amino acid sequences of equal length or between two nucleotide sequences of equal length. If the two sequences to be compared are not of equal length, they must be aligned to best possible fit. Sequence identity can, for example, be calculated by the BLAST program e.g., the BLASTP program or the BLASTN program (Pearson W. R and D. J. Lipman (1988) PNAS USA 85:2444-2448) (www.ncbl.nlm.nlh.gov/BLAST).

Preferably the immunogenic fusion protein consists of the amino acid sequences. The first amino acid sequence part consisting of 170 to 178 amino acids, preferably 174 to 175 amino acids, has at least 90% sequence identity with the full length of the amino acid sequence shown in SEQ ID NO: 7.

The second amino acid sequence part consisting of 170 to 178 amino acids, preferably 174 to 175 amino acids, has at least 90% sequence identity with the full length of the amino acid sequence shown in SEQ ID NO: 7.

The first amino acid sequence part should consist of at the most 178 amino acids, such as 170 to 178 amino acids, preferably 174 to 175 amino acids. As noted, SEQ ID NO: 7 contains 175 amino acids. The first M in the sequence is optional, thus resulting in 174 amino acids in these cases.

The first amino acid sequence part has at least 90%, such as at least 95%, such as at least 97%, at least 98%, at least 99%, or 100% sequence identity with the amino acid sequence shown in SEQ ID NO: 7.

The second amino acid sequence part should consist of at the most 178 amino acids, such as 170 to 178 amino acids, preferably 174 to 175 amino acids. As noted above, SEQ ID NO: 7 contains 175 amino acids. The first M in the sequence may be optional, thus resulting in 174 amino acids in these cases.

The second amino acid sequence part has at least 90%, such as at least 95%, such as at least 97%, at least 98%, at least 99%, or 100% sequence identity with the amino acid sequence shown in SEQ ID NO: 7.

The linker amino acid sequence part is optional. In other words, the linker amino acid sequence part, if included, may consist of 0 to 20 amino acids. The amino acids in the linker amino acid sequence part may be selected randomly, i.e., to obtain a random coil formation. The linker amino acid sequence part, if present, separates the first amino acid sequence part from the second amino acid part. In other words, the linker amino acid sequence part is provided between the first amino acid sequence part from the second amino acid part.

Either of the first and second amino acid sequence parts may be provided with a leading/trailing amino acid sequence part at the end furthest away from the other of the first and second amino acid sequence parts. The leading/trailing amino acid sequence part may comprise one or more amino acids.

The immunogenic fusion protein preferably consists of 340 to 376 amino acids, preferably 348 to 370 amino acids, more preferably 349 to 351 amino acids.

In a preferred embodiment of the immunogenic fusion protein according to the first aspect of the proposed technology:
- the first amino acid sequence part comprises, or preferably consists of, amino acids 1-175 or 2-175 of the sequence shown in SEQ ID NO: 7, and
- the second amino acid sequence part comprises, or preferably consists of, amino acids 1-175 or 2-175 of the sequence shown in SEQ ID NO: 7.

SEQ ID NO: 7 contains 175 amino acids. Amino acids 2-175 correspond to the sequence of amino acids in SEQ ID NO: 7 except for the first methionine (M). This is a preferred embodiment as the sequence according to SEQ ID NO: 7 is the most similar to the N-terminal region of the Rib protein. Furthermore, the N-terminal methionine is often cleaved off from a functional protein and thus of less importance as a recognizable component of said functional protein.

In a preferred embodiment, the immunogenic fusion protein according to the first aspect of the proposed technology, wherein the amino acid sequence comprises the linker amino acid sequence part, and wherein the linker amino acid sequence part consists of 1 to 10 amino acids, preferably 2 to 5 amino acids, more preferably 2 amino acids, and most preferably the amino acid sequence EF.

When the linker amino acid sequence part consists of the amino acids EF (Glutamate-Phenylalanine), then it is preferable that the first amino acid sequence and the second amino acid sequence part comprises, or preferably consists of, amino acids 2-175 of the sequence shown in SEQ ID NO: 7. A fusion protein comprising an amino acid linker is advantageous in that it enables modification of the characteristics of the fusion protein, such as flexibility, rigidity, or spatially separating the first amino acid sequence and the second amino acid to expose certain domains of the fusion protein.

Preferably the immunogenic fusion protein according to the first aspect of the proposed technology, wherein the immunogenic fusion protein has at least 90% sequence identity to SEQ ID NO: 51. More preferably the immunogenic fusion protein has at least 90%, such as at least 95%, such as at least 97%, at least 98%, at least 99%, or 100% sequence identity with the amino sequence shown in SEQ ID NO: 51. This is preferred as the sequence proposed in SEQ ID NO: 51 comprises two amino acid sequences corresponding to the N-terminal region of Rib protein while linked by a linker amino acid sequence comprising of Glutamate-Phenylalanine (EF). Such a fusion protein is believed to have an increased immunogenicity towards Rib compared to previously mentioned embodiments.

In a preferred embodiment the immunogenic fusion protein consists of the sequence of amino acids shown in SEQ ID NO: 51, or alternatively, wherein the immunogenic fusion protein consists of the amino acids 2-352 of SEQ ID NO: 51. Such an immunogenic fusion protein is preferred since it only contains the essential sequences of the N-terminal region of Rib coupled by a short two amino acid long linker amino acid sequence. Furthermore, the first methionine is less important as a recognizable component of the fusion protein as previously mentioned, wherein removing this further shortens the immunogenic fusion protein.

Such an immunogenic fusion protein is markedly different compared to the immunogenic fusion protein disclosed in WO 2022207657 and has a higher efficacy in eliciting immunogenicity towards Rib proteins of group B Streptococcus.

SEQ ID NO: 51 corresponds to the amino acid sequence:
MAEVISGSAVTLNTNMTKNVQNGRAYIDLYDVKNGKIDPLQLITLNSPDLK
AQYVIRQGGNYFTQPSELTTVGAASINYTVLKTDGSPHTKPDGQVDIINVSL
TIYNSSALRDKIDEVKKKAEDPKWDEGSRDKVLISLDDIKTDIDNNPKTQSD
IANKITEVTNLEKILVPRIPEFMAEVISGSAVTLNTNMTKNVQNGRAYIDLY
DVKNGKIDPLQLITLNSPDLKAQYVIRQGGNYFTQPSELTTVGAASINYTVL
KTDGSPHTKPDGQVDIINVSLTIYNSSALRDKIDEVKKKAEDPKWDEGSRD
KVLISLDDIKTDIDNNPKTQSDIANKITEVTNLEKILVPRIP

The difference between the fusion protein according to SEQ ID NO: 51 and the sequence given for the fusion protein of WO 2022207657 is the substitution of the N-terminal region sequence of the AlpC protein with a further N-terminal region sequence of the Rib protein.

In a further embodiment of the immunogenic fusion protein according to the first aspect of the proposed technology the immunogenic fusion protein is modified by glycosylation, amidation, carboxylation or phosphorylation, or by being conjugated to a capsular polysaccharide or an RSV antigen as described with regard to the third aspect of the proposed technology further below.

This is advantageous as such fusion proteins may have enhanced immunogenicity compared to unmodified immunogenic proteins. Numerous techniques are available and well known to those of skill in the art which may be used, without undue experimentation, to increase the immunogenicity of the fusion protein. For example, the fusion protein may be modified by coupling to dinitrophenol groups or arsanilic acid, or by denaturation with heat and/or SDS. Alternatively, the fusion protein may be coupled to an immunogenic carrier. For a review of some general considerations in coupling strategies, see Antibodies, A Laboratory Manual, Cold Spring Harbor Laboratory, ed. E. Harlow and D. Lane (1988). Useful immunogenic carriers are well known in the art. Examples of such carriers are keyhole limpet hemocyanin (KLH); albumins such as bovine serum albumin (BSA) and ovalbumin, PPD (purified protein derivative of tuberculin); red blood cells; tetanus toxoid; cholera toxoid; agarose beads; activated carbon; or bentonite.

In a further embodiment the immunogenic fusion protein is conjugated to a capsular polysaccharide, preferably a bacterial polysaccharide, more preferably a group B Streptococcus polysaccharide.

By polysaccharide is meant any linear or branched polymer consisting of monosaccharide residues, usually linked by glycosidic linkages, and thus includes oligosaccharides. Preferably, the polysaccharide will contain between 2 and 50 monosaccharide units, more preferably between 6 and 30 monosaccharide units. The polysaccharide component may be based on or derived from polysaccharide components of the polysaccharide capsule from many gram-positive and gram-negative bacterial pathogens such as *H. influenzae, N. meningitidis* and S. *pneumoniae.* Other bacteria from which polysaccharide components may be conjugated to the fusion proteins of the proposed technology include *Staphylococcus aureus, Klebsiella, Pseudomonas, Salmonella typhi, Pseudomonas aeruginosa,* and *Shigella dysenteriae.* Polysaccharide components suitable for use according to this aspect of the proposed technology include the Hib oligosaccharide, lipopolysaccharide from *Pseudomonas aeruginosa,* lipopolysaccharides from Salmonella, and the O-specific polysaccharide from *Shigella dysenteriae.*

Other polysaccharide components suitable for use in accordance with the proposed technology will be well-known to those skilled in the art.

Fragments of bacterial capsular polysaccharide may be produced by any suitable method, such as by acid hydrolysis or ultrasonic irradiation. Other methods of preparation of the polysaccharide components will be well known to those of skill in the art.

The polysaccharide is preferably a capsular polysaccharide derived from group B Streptococcus, or their equivalents.

The polysaccharide should preferably be coupled to the fusion protein by a covalent linkage. A particularly preferred method of coupling polysaccharide and protein is by reductive amination. Other methods include: activation of the polysaccharide with cyanogen bromide followed by reaction with adipic acid dihydrazide (spacer) and by conjugation to carboxide groups of carrier protein using soluble carbodiimides; functionalisation of the carrier protein with adipic acid dihydrazide followed by coupling to cyanogen bromide activated polysaccharides, chemical modification of both the carrier protein and the polysaccharide followed by their coupling.

The polysaccharide molecule may be coupled to the fusion protein by a spacer molecule, such as adipic acid. This spacer molecule can be used to facilitate the coupling of fusion protein to polysaccharide. After the coupling reaction has been performed, the conjugate may be purified by diafiltration or other known methods to remove unreacted protein or polysaccharide components.

If the polysaccharide is derived from a bacterial pathogen different from GBS, the conjugate may elicit immunity against two or more pathogens, e.g., multiple types of bacteria. This is a potentially important application of the immunogenic fusion protein.

A second aspect of the proposed technology concerns a nucleic acid molecule comprising a polynucleotide encoding the immunogenic fusion protein according to the first aspect of the proposed technology.

Such a nucleic acid molecule mey be used in the manufacture of the immunogenic fusion protein directly, e.g., in a cell culture from which the immunogenic fusion protein is separated and obtained, or indirectly, e.g., in a subject in which the immunogenic fusion protein is to be expressed for immunization.

The nucleic acid molecule can e.g., be a DNA molecule or an RNA molecule. The RNA molecule may be a mRNA molecule.

The nucleic acid molecule may comprise, or preferably consist of, a first polynucleotide having at least 90%, such as at least 95%, at least 96%, at least 97%, at least 98% or at least 99%, or 100%, sequence identity to one of SEQ ID NOs: 1-6 fused directly, or via a linker polynucleotide sequence, to a second polynucleotide having at least 90%, such as at least 95%, at least 96%, at least 97%, at least 98% or at least 99%, or 100%, sequence identity to one of SEQ ID NOs: 1-6.

The linker polynucleotide sequence is optional and included if the immunogenic fusion protein encoded by the nucleic acid molecule is to have a linker provided between the first amino acid sequence part and the second amino acid sequence part. If the first polynucleotide is fused directly to the second polynucleotide, then there is no linker polynucleotide sequence present in the nucleic acid molecule. Each of the SEQ ID NOs: 1-6 referred to above may be optionally truncated by removing the first three bases coding for the initial methionine (M) in the first amino acid sequence part, and the initial methionine (M) in the second amino acid sequence part of the immunogenic fusion protein encoded by the nucleic acid molecule.

The nucleic acid molecule may optionally comprise one or more stop codons.

The nucleic acid molecule may optionally comprise one or more start codons.

The nucleic acid molecule may be codon optimized for expression in a specific organism, such as *E. coli,* or *Cricetulus griseus* (Chinese Hamster Ovary) cells.

In a preferred embodiment of the nucleic acid molecule according to the second aspect of the proposed technology, the nucleic acid molecule has at least 90% sequence identity with the sequence of nucleotides shown in one of SEQ ID NOs: 45-50.

Such nucleic acid molecules are preferred because they encode the amino acid sequence as described according to the first aspect of the proposed technology having an increased immunogenicity towards Rib. A further advantage of the nucleotide sequences (SEQ ID Nos: 45-50) is that they are codon optimized for expression in several organisms, facilitating transcription and/or translation, and thus production of said nucleic acid and/ or amino acid according to the first aspect of the proposed technology.

Preferably the nucleic acid molecule has at least at least 95%, at least 96%, at least 97%, at least 98% or at least 99%, or 100%, sequence identity with the sequence of nucleotides shown in one of SEQ ID NOs: 45-50. Each of the SEQ ID NOs: 45-50 referred to in these preferred embodiments may be optionally truncated by removing the first three bases coding for the initial methionine (M) in the first amino acid sequence part of the immunogenic fusion protein encoded by the nucleic acid molecule. Further, each of the SEQ ID NOs: 45-50 referred to in these preferred embodiments may be optionally truncated by removing the last 1 to 6 bases of the two stop codons. This may for example be used when the nucleic acid molecule is to be used as a DNA or RNA vaccine - transcription and/or translation will cease at the end of the nucleic acid molecule.

The nucleic acid molecule may preferably consist of 1053 (without initial M) to 1056 bases (with initial M).

Nucleic acid DNA molecules encoding the amino acid sequence of SEQ ID NO: 51, are shown in SEQ ID NOs: 45, 47 and 49, where SEQ ID NO: 47 is codon optimized for expression in *E. coli,* and SEQ ID NO: 49 is codon optimized for expression in *Cricetulus griseus* (Chinese Hamster Ovary) cells.

Corresponding nucleic acid RNA molecules encoding the amino acid sequence of SEQ ID NO: 51 are shown in SEQ ID NOs: 46, 48 and 50, where SEQ ID NO: 48 is codon optimized for expression in *E. coli,* and SEQ ID NO: 20 is codon optimized for expression in *Cricetulus griseus* (Chinese Hamster Ovary) cells.

A third aspect of the proposed technology concerns a vector comprising the nucleic acid molecule according to the second aspect of the proposed technology.

A wide variety of expression host/vector combinations may be employed in expressing the nucleic acid molecule. Useful expression vectors for eukaryotic hosts include, for example, vectors comprising expression control sequences from SV40, bovine papilloma virus, adenovirus, adeno-associated virus, cytomegalovirus, and retroviruses. Useful expression vectors for bacterial hosts include bacterial plasmids, such as those from *E. coli,* including pBluescript, pGEX2T, pUC vectors, col E1, pCR1, pBR322, pMB9 and their derivatives, wider host range plasmids, such as RP4, phage DNAs, e.g., the numerous derivatives of phage lambda, e.g., lambda GT10 and lambda GT11, NM989, and other DNA phages, such as M13 and filamentous single stranded DNA phages. Useful expression vectors for yeast cells include the 2.mu. plasmid and derivatives thereof. Useful vectors for insect cells include pVL 941.

In addition, any of a wide variety of expression control sequences may be used in these vectors to express the nucleic acid molecules. Useful expression control sequences include the expression control sequences associated with structural genes of the foregoing expression vectors. Examples of useful expression control sequences include, for example, the early and late promoters of SV40 or adenovirus, the lac system, the trp system, the TAC or TRC system, the T3 and T7 promoters, the major operator and promoter regions of phage lambda, the control regions of fd coat protein, the promoter for 3-phosphoglycerate kinase or other glycolytic enzymes, the promoters of acid phosphatase, e.g., Pho5, the promoters of the yeast alpha-mating system and other constitutive and inducible promoter sequences known to control the expression of genes of prokaryotic or eukaryotic cells or their viruses, and various combinations thereof.

A fourth aspect of the proposed technology concerns a host cell expressing the immunogenic fusion protein according to the first aspect of the proposed technology or comprising the nucleic acid molecule according to the second aspect of the proposed technology or the vector according to the third aspect of the proposed technology.

The host cell may be a gram-negative bacterial cell, gram-positive bacterial cell, yeast cell, insect cell, animal cell, African green monkey cell, human cell, or plant cell. The host cell may be selected from the group consisting of *E. coli, Pseudomonas, Bacillus, Streptomyces, aspergillus, lactobacillus, shigella, salmonella, listeria, streptococcus, staphylococcus* and fungi. *E. coli* is however preferred.

Additionally, insect cells such as *Spodoptera frugiperda* (SF9), animal cells such as CHO and mouse cells, African green monkey cells such as COS 1, COS 7, BSC 1, BSC 40, and BMT 10, human cells, and plant cells in tissue culture, may be used. Preferred host organisms include bacteria such as *E. coli* and *B. subtilis,* and mammalian cells in tissue culture.

When a host cell is used to produce the immunogenic fusion protein, or used to produce the first and second amino acid sequence parts thereof, or used to produce the linker amino acid sequence part, then the produced amino acid sequence parts may be isolated from the microbial culture or cell culture and purified using any of a variety of conventional methods including: liquid chromatography such as normal or reversed phase, using HPLC, FPLC and the like; affinity chromatography (such as with inorganic ligands or monoclonal antibodies); ion exchange chromatography, size exclusion chromatography; immobilized metal chelate chromatography; gel electrophoresis; tangential flow filtration and the like. One of skill in the art may select the most appropriate isolation and purification techniques without departing from the scope of the proposed technology.

In addition, the immunogenic fusion protein, the first and second amino acid sequence parts thereof, or the linker amino acid sequence part, may be generated by any of several chemical techniques. For example, they may be prepared using the solid-phase synthetic technique or they may be prepared by synthesis in solution.

A fifth aspect of the proposed technology concerns a vaccine comprising one or more of the immunogenic fusion protein according to the first aspect of the proposed technology, the nucleic acid molecule according to the second aspect of the proposed technology, the vector according to the third aspect of the proposed technology, or the host cell according to the fourth aspect of the proposed technology.

The amount of each of the immunogenic fusion protein, the nucleic acid molecule, the vector, and/or the host cell is preferably a pharmaceutically effective amount, or an amount capable of eliciting protective immunity against group B Streptococcus, or an amount sufficient to elicit an immune response. Further, the amount may be such as to be sufficient to reduce and most preferably prevent a clinically significant deficit in the activity and response of the subject receiving the vaccine. Alternatively, the amount is sufficient to cause an improvement in a clinically significant condition in a host or subject.

As is appreciated by those skilled in the art, the amount of a compound may vary depending on its specific activity. Suitable dosage amounts may contain a predetermined quantity of active composition calculated to produce the desired therapeutic effect in association with the required diluents; i.e., carrier, or additive. Further, the dosage to be administered will vary depending on the active principle or principles to be used, the age, weight, etc. of the individual to be treated.

The vaccine is preferably capable of eliciting protective immunity against group B Streptococcus.

The immunogenic fusion protein elicits an immune response when administered to subjects. Such an immune response can also be obtained, indirectly, by administering the nucleic acid molecule either as a DNA nucleic acid molecule for later transcription and translation for expressing the immunogenic fusion protein, or as an RNA or mRNA nucleic acid molecule for translation and expression of the immunogenic fusion protein. The nucleic acid molecule may be provided in the vector for increased transcriptional and/or translational control, and the vector can in turn be provided in the host cell for further controlling and/or increasing the expression of the immunogenic fusion protein.

The vaccine may further comprise a pharmaceutically acceptable vehicle, and optionally an adjuvant. The term "pharmaceutical acceptable vehicle" is intended to mean any suitable acceptable excipient, adjuvants, carrier, diluent commonly used in pharmaceutical formulations.

The vaccine may encompass a vaccine composition.

The vaccine may additionally comprise other pharmacologically acceptable ingredients such as salts, buffers, immunoactive components, adjuvants (AlOH), wetting agents, emulsifying and suspending agents, or sweetening, flavouring, perfuming agents, or other substances which are desirable for improving the efficacy of the composition. A composition is said to be "pharmacologically acceptable" if its administration can be tolerated by a recipient individual.

Adjuvants are substances that can be used to specifically augment a specific immune response. Normally, the adjuvant and the composition are mixed prior to presentation to the immune system, or presented separately, but into the same site of the animal or human being immunized. Adjuvants can be loosely divided into several groups based upon their composition. These groups include oil adjuvants (for example, Freund's complete and incomplete), mineral salts for example, AlK (SO₄)₂, AlNa (SO₄)₂, AlNH₄ (SO₄), AlOH, silica, kaolin, and carbon), polynucleotides (for example, poly IC and poly AU acids), and certain natural substances (for example, wax D from *Mycobacterium tuberculosis,* as well as substances found in *Corynebacterium parvum,* or *Bordetella pertussis,* and members of the genus *Brucella.*

Alhydrogel (e.g., Aluminum hydroxide, AlOH) is an especially effective adjuvant for the immunogenic fusion protein according to the first aspect of the proposed technology and therefore especially useful in the vaccine according to the fifth aspect of the proposed technology.

Methods for the preparation and formulation of vaccines and vaccine compositions are well known to those skilled in the art. The choice of ingredients will for instance vary depending on the administration route of the composition. For example, compositions for parenteral administration include sterile aqueous or non-aqueous solutions, suspensions, and emulsions. Examples of non-aqueous solvents are propylene glycol, polyethylene glycol, vegetable oils such as olive oil, and injectable organic esters such as ethyl oleate. Carriers or occlusive dressings can be used to increase skin permeability and enhance antigen absorption. Liquid dosage forms for oral administration may generally comprise a liposome solution containing the liquid dosage form. Suitable forms for suspending liposomes include emulsions, suspensions, solutions, syrups, and elixirs containing inert diluents commonly used in the art, such as purified water.

The vaccine may be administered to humans or animals, including mammals and birds, such as rodents (mouse, rat, guinea pig, or rabbit); birds (turkey, hen or chicken); other farm animals (cow, horse, pig or piglet); pets (dog, cat and other pets); and humans. While many animals may be treated with the vaccine of the proposed technology, a preferred individual for treatment is a human or commercially valuable animal and livestock such as fish, e.g., Tilapia, and camels.

The vaccine can be administered to an individual according to methods known in the art. Such methods comprise application e.g., parenterally, such as through all routes of injection into or through the skin: e.g., intramuscular, intravenous, intraperitoneal, intradermal, mucosal, submucosal, or subcutaneous. Also, they may be applied by topical application as a drop, spray, gel or ointment to the mucosal epithelium of the eye, nose, mouth, anus, or vagina, or onto the epidermis of the outer skin at any part of the body. Other possible routes of application are by spray, aerosol, or powder application through inhalation via the respiratory tract. In this last case the particle size that is used will determine how deep the particles will penetrate into the respiratory tract. Alternatively, application can be via the alimentary route, by combining with the food, feed or drinking water e.g., as a powder, a liquid, or tablet, or by administration directly into the mouth as a: liquid, a gel, a tablet, or a capsule, or to the anus as a suppository.

Many different techniques exist for the timing of the immunizations. It is possible to administer the vaccine or the components a-d (immunogenic fusion protein according to the first aspect, nucleic acid molecule according to the second aspect, vector according to the third aspect, host cell according to the fourth aspect) more than once to increase the levels and diversities of expression of the immunoglobulin repertoire expressed by the immunized animal. Typically, if multiple immunizations are given, they may be given a week to three months apart, such as two weeks to two months apart, such as one to two months apart, or four weeks apart. Additionally, one or maybe more booster injections of the vaccine or the components a-d may be administered. Preferably, booster injections may be administered at about 1 and 6 months after the initial injection or injections.

The vaccine according to the fifth aspect of the proposed technology may preferably comprise one or more additional immune active components. The additional immunoactive component may be an antigen, an immune enhancing substance, and/or a vaccine; either of these may comprise an adjuvant.

Accordingly, in a preferred embodiment of the vaccine according to the fifth aspect of the proposed technology, the vaccine additionally comprises one or more of:
a) a first further immunogenic fusion protein comprising or consisting of an amino acid sequence having at least 90%, such as 95, 96, 97, 98 or 99 % sequence identity with the amino acid sequence shown in SEQ ID NO: 21;
b) a first further nucleic acid molecule encoding the further immunogenic fusion protein, the further nucleic acid molecule preferably having at least 90% sequence identity with the sequence of nucleotides shown in one of SEQ ID NOs: 15-20;
c) a second further immunogenic fusion protein comprising or consisting of an amino acid sequence having at least 90%, such as 95, 96, 97, 98 or 99 % sequence identity with the amino acid sequence shown in SEQ ID NO: 42;
d) a second further nucleic acid molecule encoding the further immunogenic fusion protein, the further nucleic acid molecule preferably having at least 90% sequence identity with the sequence of nucleotides shown in one of SEQ ID NOs: 36-41.

As will be discussed further below, the first further immunogenic fusion protein is derived from the N-terminal regions of the GBS surface proteins Rib and AlpC, as also disclosed in WO 2022207657. The second further immunogenic fusion protein is derived from the N-terminal regions of the GBS surface proteins Alp1 and Alp2/3, as also disclosed in WO 2022207657.

Further, by including one or more additional component of a-d, the vaccine can provide a broader coverage of protection against all Group B Streptococcus strains. Similar to as described above for the immunogenic fusion protein, the first and/ or second further immunogenic fusion protein may be expressed indirectly by the further nucleic acid molecule, further vector, and the further host cell.

The amount of each of a-d should preferably be a pharmaceutically effective amount, or an amount capable of eliciting protective immunity against group B Streptococcus, or an amount sufficient to elicit an immune response, or at least an amount sufficient to increase the general immune response to the vaccine.

Furthermore, as noted above, the first further immunogenic fusion protein has at least 90%, such as 95, 96, 97, 98 or 99 % sequence identity with the amino acid sequence shown in SEQ ID NO: 21 which SEQ ID NO: 21 represents the following amino acid sequence:

MAEVISGSAVTLNTNMTKNVQNGRAYIDLYDVKNGKIDPLQLITLNSPDLK
AQYVIRQGGNYFTQPSELTTVGAASINYTVLKTDGSPHTKPDGQVDIINVSL
TIYNSSALRDKIDEVKKKAEDPKWDEGSRDKVLISLDDIKTDIDNNPKTQSD
IANKITEVTNLEKILVPRIPEFSTIPGSAATLNTSITKNIQNGNAYIDLYDVKL
GKIDPLQLIVLEQGFTAKYVFRQGTKYYGDVSQLQSTGRASLTYNIFGEDGL
PHVKTDGQIDIVSVALTIYDSTTLRDKIEEVRTNANDPKWTEESRTEVLTGL
DTIKTDIDNNPKTQTDIDSKIVEVNELEKLLVLS

Nucleic acid DNA molecules encoding the amino acid sequence of SEQ ID NO: 21, i.e., as encompassed by (b) are shown in SEQ ID NOs: 15, 17 and 19, where SEQ ID NO: 17 is codon optimized for expression in *E. coli,* and SEQ ID NO: 19 is codon optimized for expression in *Cricetulus griseus* (Chinese Hamster Ovary) cells.

Corresponding nucleic acid RNA molecules encoding the amino acid sequence of SEQ ID NO: 21 are shown in SEQ ID NOs: 16, 18 and 20, where SEQ ID NO: 18 is codon optimized for expression in *E. coli,* and SEQ ID NO: 20 is codon optimized for expression in *Cricetulus griseus* (Chinese Hamster Ovary) cells.

As mentioned above, the first further immunogenic fusion protein is derived from the N-terminal regions of the GBS surface proteins Rib and AlpC.

The group B Streptococcus Rib protein, also referred to in this specification as Rib and Rib protein, has been described and characterized above. SEQ ID NO: 7 shows the N-terminal Region of Rib used in the fusion protein.

The group B Streptococcus Alpha C protein, herein referred to AlpC, is a group B Streptococcus surface protein known in the art. WO 9410317 describes a conjugate vaccine composition comprising the alpha protein. The native group B Streptococcus AlpC precursor protein as described in WO 9410317 has a molecular weight of 108 kDa. Cleavage of the putative signal sequence of 41 amino acids yields a mature protein of 104 kDa. (Note, however, that the signal sequence was subsequently shown to have a length of 56 amino acid residues: Stålhammar-Carlemalm et al., J Exp Med 177,1593; 1993). The 20 kDa N-terminal region of the AlpC antigen shows no homology to previously described protein sequences and is followed by a series of nine tandem repeating units that make up 74% of the mature protein. Each repeating unit (denoted herein as "R") is identical and consists of 82 amino acids with a molecular mass of about 8500 Daltons, which is encoded by 246 nucleotides. The C-terminal region of the AlpC antigen contains a cell wall anchor domain motif present in a number of gram-positive surface proteins.

SEQ ID NO: 14 shows the shorter N-terminal Region of AlpC:
STIPGSAATLNTSITKNIQNGNAYIDLYDVKLGKIDPLQLIVLEQGFTAKYVF
RQGTKYYGDVSQLQSTGRASLTYNIFGEDGLPHVKTDGQIDIVSVALTIYDS
TTLRDKIEEVRTNANDPKWTEESRTEVLTGLDTIKTDIDNNPKTQTDIDSKIV
EVNELEKLLVLS

The difference between the shorter N-terminal region of AlpC, i.e. SEQ ID NO: 14, and the sequence given for the N-terminal of AlpC in WO 2008127179 is the 5 amino acid long leading sequence GPLGS, SEQ ID NO: 44.

Nucleic acid DNA molecules encoding the amino acid sequence of SEQ ID NO: 14 are shown in SEQ ID NOs: 8, 10, 12, where SEQ ID NO: 10 is codon optimized for expression in *E. coli,* and SEQ ID NO: 12 is codon optimized for expression in *Cricetulus griseus* (Chinese Hamster Ovary) cells.

Corresponding nucleic acid RNA molecules encoding the amino acid sequence of SEQ ID NO: 14 are shown in SEQ ID NOs: 9, 11, 13, where SEQ ID NO: 11 is codon optimized for expression in *E. coli,* and SEQ ID NO: 13 is codon optimized for expression in *Cricetulus griseus* (Chinese Hamster Ovary) cells.

The first further immunogenic fusion protein may thus comprise or consist of an amino acid sequence consisting of:
i. a further first amino acid sequence part having at least 90% sequence identity with the amino acid sequence shown in SEQ ID NO: 7;
ii. a further second amino acid sequence part having at least 90% sequence identity with the amino acid sequence shown in SEQ ID NO: 14; and optionally:
iii. a further linker amino acid sequence part consisting of 1 to 20 amino acids and separating the further first amino acid sequence part from the further second amino acid part.

As noted above, the second further immunogenic fusion protein has at least 90%, such as 95, 96, 97, 98 or 99 % sequence identity with the amino acid sequence shown in SEQ ID NO: 42, which SEQ ID NO: 42 represents the following amino acid sequence:

MAEVISGSAATLNSALVKNVSGGKAYIDIYDVKNGKIDPLNLIVLTPSNYSA
NYYIKQGGRIFTSVNQLQTPGTATITYNILDENGNPYTKSDGQIDIVSLVTTV
YDTTELRNNINKVIENANDPKWSDDSRKDVLSKIEVIKNDIDNNPKTQSDID
NKIVEVNELEKLLVLPEFSTIPGSAATLNTSITKNIQNGNAYIDLYDVKNGLI
DPQNLIVLNPSSYSANYYIKQGAKYYSNPSEITTTGSATITFNILDETGNPHK
KADGQIDIVSVNLTIYDSTALRNRIDEVINNANDPKWSDGSRDEVLTGLEKI
KKDIDNNPKTQIDIDNKINEVNEIEKLLVVSL

Nucleic acid DNA molecules encoding the amino acid sequence of SEQ ID NO: 42, i.e., as encompassed by (b) are shown in SEQ ID NOs: 36, 38, and 40, where SEQ ID NO: 38 is codon optimized for expression in *E. coli,* and SEQ ID NO: 40 is codon optimized for expression in *Cricetulus griseus* (Chinese Hamster Ovary) cells.

Corresponding nucleic acid RNA molecules encoding the amino acid sequence of SEQ ID NO: 42 are shown in SEQ ID NOs: 37, 39, and 41, where SEQ ID NO: 39 is codon optimized for expression in *E. coli,* and SEQ ID NO: 41 is codon optimized for expression in *Cricetulus griseus* (Chinese Hamster Ovary) cells.

As mentioned above, the second further immunogenic fusion protein is derived from the N-terminal regions of the GBS surface proteins Alp1 and Alp2/3.

The Alp1 protein is also known as epsilon protein and is a group B streptococcal alpha-protein-like protein.

The amino acid sequence of the N-terminal region of Alp1 is given by SEQ ID NO: 28:

MAEVISGSAATLNSALVKNVSGGKAYIDIYDVKNGKIDPLNLIVLTPSNYSA
NYYIKQGGRIFTSVNQLQTPGTATITYNILDENGNPYTKSDGQIDIVSLVTTV

YDTTELRNNINKVIENANDPKWSDDSRKDVLSKIEVIKNDIDNNPKTQSDID
NKIVEVNELEKLLVLP

Nucleic acid DNA molecules encoding the amino acid sequence of SEQ ID NO: 28 are shown in SEQ ID NOs: 22, 24, and 26, where SEQ ID NO: 24 is codon optimized for expression in *E. coli,* and SEQ ID NO: 26 is codon optimized for expression in *Cricetulus griseus* (Chinese Hamster Ovary) cells.

Corresponding nucleic acid RNA molecules encoding the amino acid sequence of SEQ ID NO: 28 are shown in SEQ ID NOs: 23, 25, and 27, where SEQ ID NO: 25 is codon optimized for expression in *E. coli,* and SEQ ID NO: 27 is codon optimized for expression in *Cricetulus griseus* (Chinese Hamster Ovary) cells.

The Alp2 protein is another alpha-protein-like-protein. Like the other members of the family, the Alp2 protein has an N-terminal domain and several repeated domains towards the C-terminus. The N-terminal region of Alp2 and Alp3 are identical. Alp3 is another alpha-protein-like-protein, also known as R28. It is very similar to the R28 protein also found in *S. pyogenes.*

The amino acid sequence of the N-terminal region of Alp2 is given by SEQ ID NO: 35:

STIPGSAATLNTSITKNIQNGNAYIDLYDVKNGLIDPQNLIVLNPSSYSANYYI
KQGAKYYSNPSEITTTGSATITFNILDETGNPHKKADGQIDIVSVNLTIYDST
ALRNRIDEVINNANDPKWSDGSRDEVLTGLEKIKKDIDNNPKTQIDIDNKIN
EVNEIEKLLVVSL

Nucleic acid DNA molecules encoding the amino acid sequence of SEQ ID NO: 35 are shown in SEQ ID NOs: 29, 31, and 33, where SEQ ID NO: 31 is codon optimized for expression in *E. coli,* and SEQ ID NO: 33 is codon optimized for expression in *Cricetulus griseus* (Chinese Hamster Ovary) cells.

Corresponding nucleic acid RNA molecules encoding the amino acid sequence of SEQ ID NO: 35 are shown in SEQ ID NOs: 30, 32, and 34, where SEQ ID NO: 32 is codon optimized for expression in *E. coli,* and SEQ ID NO: 34 is codon optimized for expression in *Cricetulus griseus* (Chinese Hamster Ovary) cells.

SEQ ID NO: 42 thus correspond to SEQ ID NO: 28 (N-terminal of Alp1) fused via a linker amino acid sequence part consisting of the amino acids EF to SEQ ID NO: 35 (N-terminal of Alp2/3).

The second further immunogenic fusion protein may thus comprise or consist of an amino acid sequence consisting of:
i. a further third amino acid sequence part having at least 90% sequence identity with the amino acid sequence shown in SEQ ID NO: 28;
ii. a further fourth amino acid sequence part having at least 90% sequence identity with the amino acid sequence shown in SEQ ID NO: 35; and optionally:
iii. a further linker amino acid sequence part consisting of 1 to 20 amino acids and separating the further first amino acid sequence part from the further second amino acid part,

In these embodiments there are preferably no other proteins or amino acids sequences, different from the specified immunogenic fusion protein and the specified further immunogenic fusion protein, in the vaccine.

In each of SEQ ID NO: 28 and 42, the initial M may be optional.

As such, it is preferable to exclude the initial methionine (M) from SEQ ID NO: 28 and 42 to further shorten the immunogenic fusion proteins included in the vaccine. Furthermore, it is preferable to include additional immunogenic fusion proteins to the vaccine to provide a broader coverage of protection against many Group B Streptococcus strains.

Accordingly, in a preferred embodiment of the vaccine according to the fifth aspect of the proposed technology the vaccine comprises:
a) the immunogenic fusion protein according to the first aspect of the proposed technology; and additionally comprising one or more of
b) a first further immunogenic fusion protein comprising, preferably consisting of, the sequence of amino acids shown in SEQ ID NO: 21, or alternatively comprising, preferably consisting of, the amino acids 2-347 of SEQ ID NO: 21, and
c) a second further immunogenic fusion protein comprising, preferably consisting of, the sequence of amino acids shown in SEQ ID NO: 42, or alternatively comprising, preferably consisting of, the amino acids 2-347 of SEQ ID NO: 42.

The most preferred sequence of the immunogenic fusion protein according to the first aspect of the proposed technology is shown in SEQ ID NO: 51. Accordingly, in a preferred embodiment of the vaccine according to the fifth aspect of the proposed technology the vaccine comprises:
a) the immunogenic fusion protein according to the first aspect of the proposed technology, wherein the immunogenic fusion protein consists of the sequence of amino acids shown in SEQ ID NO: 51, or alternatively, wherein the immunogenic fusion protein consists of the amino acids 2-352 of SEQ ID NO: 51; and additionally comprising one or more of
b) a first further immunogenic fusion protein comprising, preferably consisting of, the sequence of amino acids shown in SEQ ID NO: 21, or alternatively, comprising, preferably consisting of, the amino acids 2-347 of SEQ ID NO: 21;
c) a second further immunogenic fusion protein comprising, preferably consisting of, the sequence of amino acids shown in SEQ ID NO: 42, or alternatively, comprising, preferably consisting of, the amino acids 2-347 of SEQ ID NO: 42.

The most preferred embodiment of the vaccine according to the fifth aspect of the proposed technology is when vaccine comprises the most preferred immunogenic fusion protein according to the first aspect of the proposed technology and both of the further immunogenic fusion proteins according to the SEQ ID Nos: 21 and 42. Accordingly in the most preferred embodiment of the vaccine according to the fifth aspect of the proposed technology the vaccine comprises:
a) the immunogenic fusion protein according to the first aspect of the proposed technology, wherein the immunogenic fusion protein consists of the sequence of amino acids shown in SEQ ID NO: 51, or alternatively, wherein the immunogenic fusion protein consists of the amino acids 2-352 of SEQ ID NO: 51; and
b) a first further immunogenic fusion protein comprising, preferably consisting of, the sequence of amino acids shown in SEQ ID NO: 21, or alternatively, comprising, preferably consisting of, the amino acids 2-347 of SEQ ID NO: 21; and
c) a second further immunogenic fusion protein comprising, preferably consisting of, the sequence of amino acids shown in SEQ ID NO: 42, or alternatively, comprising, preferably consisting of, the amino acids 2-347 of SEQ ID NO: 42.

In these embodiments there are preferably no other proteins or amino acids sequences, different from the specified immunogenic fusion protein and the specified further immunogenic fusion protein, in the vaccine.

In a preferred embodiment of the vaccine according to the fifth aspect of the proposed technology the vaccine further comprises aluminum hydroxide as an adjuvant. This is preferred because the addition of aluminum hydroxide as an adjuvant increases the efficacy of the immunogenicity of the vaccine. In these embodiments the vaccine is preferably a pharmaceutical composition and further comprises a pharmaceutically acceptable excipient, diluent or carrier.

A sixth aspect of the proposed technology concerns the immunogenic fusion protein according to the first aspect of the proposed technology, the nucleic acid molecule according to the second aspect of the proposed technology, the vector according to the third aspect of the proposed technology, the host cell according to fourth aspect of the proposed technology, or the vaccine according to the fifth aspect of the proposed technology, for use in i) vaccination against group B Streptococcus infection, or ii) treating a group B Streptococcus infection.

The immunogenic fusion protein, the nucleic acid molecule, the vector, the host cell, or the vaccine may be used for such vaccination or treating by administering the immunogenic fusion protein, the nucleic acid molecule, the vector, the host cell, or the vaccine to a subject in need thereof.

A corresponding seventh aspect of the proposed technology concerns a method of i) vaccination against group B Streptococcus infection, or ii) treating a group B Streptococcus infection, by administrating the immunogenic fusion protein according to the first aspect of the proposed technology, the nucleic acid molecule according to the second aspect of the proposed technology, the vector according to the third aspect of the proposed technology, the host cell according to the fourth aspect of the proposed technology, or the vaccine according to the fifth aspect of the proposed technology, to a subject in need thereof.

A corresponding eight aspect of the proposed technology concerns the use of the immunogenic fusion protein according to the first aspect of the proposed technology, the nucleic acid molecule according to the second aspect of the proposed technology, the vector according to the third aspect of the proposed technology, the host cell according to fourth aspect of the proposed technology for manufacturing a pharmaceutical composition or medicament, such as a vaccine, for i) vaccination against group B Streptococcus infection, or ii) treating a group B Streptococcus infection

The immunogenic fusion protein according to the first aspect of the proposed technology, the nucleic acid molecule according to the second aspect of the proposed technology, the vector according to the third aspect of the proposed technology, the host cell according to fourth aspect of the proposed technology, or the vaccine according to the fifth aspect of the proposed technology may thus be used in a method of preventing or treating an infection caused by a group B Streptococcus.

Maternal immunoprophylaxis with a vaccine, for protecting against infection to group B Streptococcus both in the mother and in the young infant, has long been proposed as a potential route.

Thus, in preferred embodiments of the sixth, seventh and eight aspects of the proposed technology, the immunogenic fusion protein according to the first aspect of the proposed technology, the nucleic acid molecule according to the second aspect of the proposed technology, the vector according to the third aspect of the proposed technology, the host cell according to fourth aspect of the proposed technology, or the vaccine according to the fifth aspect of the proposed technology is administered to a female human subject.

The administration is thus capable of conferring immunity to GBS infection to an unborn offspring of the human female. Further, the administration may also be made to a male if the male is older.

According to this embodiment, the immunogenic fusion protein, the nucleic acid molecule, the vector, the host cell, or the vaccine is administered to a non-pregnant female or to a pregnant female, under conditions of time and amount sufficient to cause the production of antibodies which serve to protect both the female and a fetus or newborn (via passive transfer of antibodies across the placenta).

The terms "preventing or treating" in its various grammatical forms in relation to the proposed technology refer to preventing, curing, reversing, attenuating, alleviating, ameliorating, inhibiting, minimizing, suppressing, or halting (1) the deleterious effects of a disorder associated with group B Streptococcus infection, (2) disorder progression, or (3) disorder causative agent (group B Streptococcus). Further, the terms "preventing or treating" are contemplated to include the creation of total or partial immunity of the individual to group B Streptococcus infection.

A ninth aspect of the proposed technology concerns a method for preventing or treating an infection caused by a group B Streptococcus which comprises administering to an individual in need thereof an effective amount of antibodies elicited from the exposure of a second individual to the immunogenic fusion protein, the nucleic acid molecule, the vector, the host cell, or the vaccine according to the first to fifth aspects of the proposed technology.

According to this aspect, resistance or immunity to group B Streptococcus is conferred to the individual by passive immunization, i.e., the elicited antisera is recovered and directly provided to a recipient suspected of having an infection caused by a group B Streptococcus. It is contemplated that such antisera could be administered to a pregnant female (at or prior to parturition), under conditions of time and amount sufficient so that the antisera would serve to protect either the fetus or newborn (via passive incorporation of the antibodies across the placenta). The vaccine or antisera of the proposed technology may, thus, be provided either prior to the onset of infection (so as to prevent or attenuate an anticipated infection) or after the initiation of an actual infection.

A tenth aspect of the proposed technology concerns a method of producing an immunogenic fusion protein comprising the steps of
- providing a host cell according to the fourth aspect of the proposed technology,
- multiplying the host cell,
- purifying the immunogenic fusion protein according to the first aspect of the proposed technology, and
- obtaining the immunogenic fusion protein.

Alternatively, the immunogenic fusion protein can be produced in a cell-free expression system. Such systems comprise all essential factors for expression from an appropriate recombinant nucleic acid, operably linked to a promoter that will function in that particular system.

### Example 1 - Different IgG GMC for the N-terminal domains of Rib and AlpC AlpN after immunization with a fusion protein consisting said domains

In this example, AlpN domain specific IgG geometric mean concentrations (GMC) were measured after immunization with a fusion protein consisting of the N-terminal domains of Rib and AlpC, this fusion protein herein being referred to as GBS-NN.

The immunization protocol consisted of two doses of 50 mg GBS-NN plus aluminum hydroxide (AlOH). The first dose was administered on day 0 and the second dose was administered on day 28 (4 weeks later). Measurements were made pre-vaccination (day 0) and four weeks after a second vaccine dose (day 57).

The immunized subjects were healthy adult women enrolled in a two-part (IA, IB), randomized, double blind, placebo-controlled clinical phase I trial of conducted in a total of 240 healthy adult non-pregnant women.

Quantification of IgG against AlpCN and RibN was done by ELISA using human immunoglobulin for subcutaneous administration (Subcuvia, 160 mg/mL, Baxalta, Sweden) (SCIG) as a standard with known concentration of Abs specific to each protein. The SCIG preparation was calibrated for all isotypes and IgG subclasses against the individual AlpN domains based on equivalence of absorbance between a reference ELISA and the anti-AlpN ELISA performed in parallel under identical conditions. For coating, ELISA plates (Corning Costar, High Binding) were incubated overnight at 4°C with 100 ml of relevant recombinant AlpN domain per well, all diluted to 0.5 mg/mL in PBS. Pre- and post-vaccination serum from the **study subjects** was serially diluted in PBS-2% BSA-0.05% Tween 20 (sample buffer) and added to the wells (100 ml/well). Calibrated SCIG was serially diluted in sample buffer and added to separate rows of wells on each plate (100 ml/well) as a standard. After 2 h incubation at room temperature the plate was washed and horseradish peroxidase (HRP)-conjugated detection Abs diluted in the sample buffer were added to all wells (100 ml/well): goat anti-human IgG (g-chain) F(ab')2-HRP (Sigma A2290). The plate was incubated with HRP-conjugated detection Abs for 1 h at room temperature. After washing, HRP was detected using tetrametylbenzidine (TMB; Sigma T8768)-hydrogen peroxide (100 ml/well). Color reaction was stopped with 50 ml of 1 M sulfuric acid/well and the absorbance was read in SpectroStar photometer at 450 nm wavelength. Raw data were analyzed using four parameters logistic (4PL) fit function in Prism7 software (GraphPad). Concentrations of Abs in serum samples were computed for all sample dilutions using absorbance values of calibrated SCIG dilutions as standard curves. Linearity of serum sample curves was assessed by computing coefficient of variation (CV) for all sample dilutions. When the CV for at least 3 dilutions was < 20% the result was considered valid.

The results are shown below in Table 1.

**Table 1. AlpN domain specific IgG geometric mean concentrations (GMC) relative pre-vaccination baseline levels (Day 0) four weeks after a second vaccine dose (Day 57). Two separate cohorts receiving 50 µg GBS-NN plus AlOH in the clinical phase IA trial (n=8) and IB trial (n=45) are included in the analyses (n=53 in total).**

| AlpN domain | Day 0 [µg/ml] (95% CI) | Day 57 [µg/ml] (95% CI) |
|---|---|---|
| AlpCN | 0.12 (0.09 - 0.16) | 5.45 (3.71 - 8.01) |
| RibN | 0.05 (0.04 - 0.06) | 2.22 (1.67 - 2.96) |

As seen in the table, the IgG GMC for RibN at 2.22 µg/ml is less than half that of AlpCN at 5.45 µg/ml. Noting that the GBS-NN fusion protein contained equal numbers of RibN domains and AlpCN domains, it appears that RibN may be less immunogenic than AlpCN. Further, noting that a majority of GBS strains express Rib, this difference in immune response against the domains revealed the need for increased immune response against Rib. By producing a fusion protein comprising two Rib N-terminal regions, the immune response against Rib is expected to be increased. The fusion protein can then be combined with the GBS-NN fusion protein to increase and/or balance the immune response, e.g. IgG concentration, against RibN in comparison to the immune response against AlpCN.

## Claims

1. An immunogenic fusion protein comprising or consisting of an amino acid sequence consisting of:
i. a first amino acid sequence part consisting of 170 to 178 amino acids, preferably 174 to 175 amino acids, and having at least 90% sequence identity with the amino acid sequence shown in SEQ ID NO: 7;
ii. a second amino acid sequence part consisting of 170 to 178 amino acids, preferably 174 to 175 amino acids, and having at least 90% sequence identity with the amino acid sequence shown in SEQ ID NO: 7; and optionally:
iii. a linker amino acid sequence part consisting of 1 to 20 amino acids and separating the first amino acid sequence part from the second amino acid part,
and wherein the immunogenic fusion protein preferably consists of 340 to 376 amino acids, preferably 348 to 370 amino acids, more preferably 349 to 351 amino acids.

2. The immunogenic fusion protein according to claim 1, wherein:
- the first amino acid sequence part comprises, or preferably consists of, amino acids 1-175 or 2-175 of the sequence shown in SEQ ID NO: 7, and
- the second amino acid sequence part comprises, or preferably consists of, amino acids 1-175 or 2-175 of the sequence shown in SEQ ID NO: 7.

3. The immunogenic fusion protein according to any preceding claim, wherein the amino acid sequence comprises the linker amino acid sequence part, and wherein the linker amino acid sequence part consists of 1 to 10 amino acids, preferably 2 to 5 amino acids, more preferably 2 amino acids, and most preferably the amino acid sequence EF.

4. The immunogenic fusion protein according to any preceding claim, wherein the immunogenic fusion protein has at least 90% sequence identity to SEQ ID NO: 51.

5. The immunogenic fusion protein according to any preceding claim, wherein the immunogenic fusion protein consists of the sequence of amino acids shown in SEQ ID NO: 51, or alternatively, wherein the immunogenic fusion protein consists of the amino acids 2-352 of SEQ ID NO: 51.

6. A nucleic acid molecule comprising a polynucleotide encoding the immunogenic fusion protein according to any of the proceeding claims.

7. The nucleic acid molecule according to claim 6, wherein the nucleic acid molecule has at least 90% sequence identity with the sequence of nucleotides shown in one of SEQ ID NOs: 45-50.

8. A vector comprising the nucleic acid molecule according to any of the claims 6-7.

9. A host cell expressing the immunogenic fusion protein according to any of the claims 1-5 or comprising the nucleic acid molecule according to any of the claims 6-7 or the vector according to claim 8.

10. A vaccine comprising one or more of the immunogenic fusion protein according to any of the claims 1-5, the nucleic acid molecule according to any of the claim 6-7, the vector according to claim 8, or the host cell according to claim 9.

11. The vaccine according to claim 10, additionally comprising one or more of:
a) a first further immunogenic fusion protein comprising or consisting of an amino acid sequence having at least 90%, such as 95, 96, 97, 98 or 99 % sequence identity with the amino acid sequence shown in SEQ ID NO: 21;
b) a first further nucleic acid molecule encoding the further immunogenic fusion protein, the further nucleic acid molecule preferably having at least 90% sequence identity with the sequence of nucleotides shown in one of SEQ ID NOs: 15-20;
c) a second further immunogenic fusion protein comprising or consisting of an amino acid sequence having at least 90%, such as 95, 96, 97, 98 or 99 % sequence identity with the amino acid sequence shown in SEQ ID NO: 42;
d) a second further nucleic acid molecule encoding the further immunogenic fusion protein, the further nucleic acid molecule preferably having at least 90% sequence identity with the sequence of nucleotides shown in one of SEQ ID NOs: 36-41;
e) a further vector comprising the further nucleic acid molecule; or
f) a further host cell expressing the further immunogenic fusion protein, or comprising the further vector.

12. The vaccine according to claim 11, wherein the vaccine comprises:
a) the immunogenic fusion protein according to claim 5; and
b) a first further immunogenic fusion protein comprising, preferably consisting of, the sequence of amino acids shown in SEQ ID NO: 21, or alternatively comprising, preferably consisting of, the amino acids 2-347 of SEQ ID NO: 21, and
c) a second further immunogenic fusion protein comprising, preferably consisting of, the sequence of amino acids shown in SEQ ID NO: 42, or alternatively comprising, preferably consisting of, the amino acids 2-347 of SEQ ID NO: 42.

13. The vaccine according to any of the claim 10-12, further comprising aluminum hydroxide as an adjuvant.

14. The immunogenic fusion protein according to any of the claims 1-5, the nucleic acid molecule according to any of the claim 6-7, the vector according to claim 8, the host cell according to claim 9, or the vaccine according to any of the claims 10-13, for use in i) vaccination against group B Streptococcus infection, or ii) treating a group B Streptococcus infection.

15. The immunogenic fusion protein according to any of the claims 1-5, the nucleic acid molecule according to any of the claim 6-7, the vector according to claim 8, the host cell according to claim 9, or the vaccine according to any of the claims 10-13, for use according to claim 14, wherein the immunogenic fusion protein according to any of the claims 1-5, the nucleic acid molecule according to any of the claim 6-7, the vector according to claim 8, the host cell according to claim 9, or the vaccine according to any of the claims 10-13, is administered to a female human subject.
